# EUROPEAN PATENT APPLICATION

(11) **EP 4 632 055 A1**
(43) Date of publication of application: **15.10.2025**
(21) Application number: 24180585.2
(22) Date of filing: 06.06.2024
(51) Int. Cl.: C11D 3/48, A61K 8/02, A61Q 17/00, C11D 3/20, C11D 17/04, C11D 1/62

(54) **WET WIPES WITH IMPROVED PRESERVATIVE PROPERTIES**

(30) Priority: 08.04.2024 US 202418629336
(71) Applicant: Rockline Industries, Inc., Sheboygan, WI 53081 (US)
(72) Inventor: Chandarana, Ravi, Leicester, LE4 3LA (GB); Cole, Douglas B., Belgium, WI 53004 (US)
(74) Representative: Barker Brettell LLP

(57) **Abstract**

A cleaning composition including a preservative formulation having a lactic acid salt, a gluconic acid salt, a polyhydric alcohol and optionally a phospholipid complex is disclosed. The cleaning composition can be loaded on a cleaning wipe and used for personal care. The lactic acid salt, gluconic acid salt and polyhydric alcohol provide a synergistic preservative effect to a cleaning composition incorporating the preservative formulation.

## Description

### Field of the Invention

The present invention relates to a cleaning composition comprising one or more lactate compounds, an alcohol, a glucose derivative and optionally a phospholipid complex.

### Background of the Invention

Increasing numbers of consumers are seeking cleaning products that not only are more natural or sustainable, but which also exhibit better overall safety of use. Consumers prefer products that can be readily used around children and pets in convenient forms such as pre-loaded disposable wipes or ready to use sprays, but these safer and more sustainable products still are expected to deliver performance on many attributes, such as cleaning and reduction of germs, at parity to traditional products.

In particular, wet wipes, or cleaning wipes, have gained wide public acceptance in the area of infant care products. Infant care wipes commonly include mild cleaning solutions, while facial wipes can include emulsions (i.e. water-in-oil or oil-in-water). Cleaning wipes can also include waxes and polish to clean furniture and/or other metal, plastic and/or wood surfaces. Wet wipes can further include soaps and/or detergents to clean an individual's hands, countertops, floors, appliances, and/or the like. An additional ingredient is ammonia for cleaning glass surfaces. Short chain alcohols and various other biocides can also be included on cleaning wipes to disinfect or sanitize a variety of surfaces.

Traditional wet wipe preservation systems used in personal care applications continue to present challenges to effectively control microbial growth, which is inherent and part of the natural bioburden that occurs in raw materials and during manufacturing, storage and consumer use. Many preservative ingredients and compositions found in wet wipes have been used for many years but do not provide sufficient antimicrobial properties required for current commercial products. In addition, many of the prior art preservative ingredients and compounds are no longer regulatory compliant, fail to work effectively and/or are not acceptable to consumers for a variety of reasons.

In view of the present state of the art of cleaning compositions for wet wipes, there is a need for an improved preservative composition for inclusion within a cleaning formulation or composition that is loaded onto a wet wipe that can be used in a variety of applications related to personal care, surface cleaning, antibacterial, disinfects, sanitizers, and/or surfaces without the deficiencies presented above.

### Summary of the Invention

In light of the foregoing, in one exemplary embodiment the invention is directed to an effective self-preservation system or broad-spectrum antimicrobial enhancement activity or preservative composition for use in conjunction with or as a part of cleaning compositions applied to and/or impregnated within wet wipes. The self-preservation system or broad-spectrum antimicrobial enhancement activity/preservative composition is a formulation that comprises one or more ingredients heretofore not recognized for having microorganism inhibiting/preservative/antimicrobial properties, including one or more lactate compounds in combination with a glucose derivative, a polyhydric alcohol and optionally a phospholipid complex, such as dimonium chloride phosphates, including those with a fatty acid chain length between 8 and 20 carbons, including but not limited to cocamidopropyl PG-dimonium chloride phosphate and other alkyl PG-dimonium chloride phosphates. Self-preservation is achieved by combining previously unknown preservative-functional ingredients that collectively produce an environment that is unfavorable for the growth of microorganisms in a synergistic manner that provides unexpected results in view of the respective inabilities of the individual components to inhibit microorganism growth. Further, the components of the preservative composition provide the unexpected preservative function to the cleaning composition and wet wipe in an environmentally-friendly manner.

According to another exemplary embodiment of the invention, other components are optionally present in a cleaning composition including the preservative formulation, such as, for example, water, anti-microbial components, surfactants, emollients, solvents, skin conditioning agents, humectants, fragrances, botanical extracts, oils, silicones and the like, and combinations of the same. All components of the formulation are blended together to form a wet wipe composition, solution or emulsion that can be impregnated within or otherwise applied to a nonwoven, cloth or paper substrate, or combinations of the same to form a usable wet wipe that is sufficiently preserved for storage and consumer use.

According to one aspect of an exemplary embodiment of the invention, a method of cleaning a surface includes the steps of providing a cleaning composition having a preservative composition including at least one lactate compound, a glucose derivative, a diol and optionally an organic phospholipid and applying the cleaning composition to surface.

According to still another aspect of an exemplary embodiment off the invention, a cleaning wipe includes a substrate and a cleaning composition loaded onto the substrate, the cleaning composition formed of preservative composition having at least one lactate compound, a glucose derivative, a diol and optionally an alkyl PG-dimonium chloride phosphate.

According to still a further aspect of an exemplary embodiment of the invention, a cleaning composition includes a preservative composition having at least one lactate compound, a glucose derivative, a diol and optionally an organic phospholipid for use as a preservative and/or anti-microbial component of the cleaning composition.

Without limitation then, in other exemplary embodiments this invention is also a wet wipe, or cleaning wipe, comprising a formulation comprising at least one layer of a nonwoven material, and a cleaning composition with a preservative component comprising at least one lactate compound, a glucose derivative, one or more alcohols, such as one or more diols, and optionally an alkyl PG-dimonium chloride phosphate with a fatty acid chain length between 8 and 20 carbons. In one aspect, other components are optionally present in the cleaning composition, such as, for example, water, surfactants, emollients, solvents, skin conditioning agents, humectants, fragrances, botanical extracts, oils, silicones and the like, and combinations of the same.

Other features, benefits and advantages of the present invention will be apparent from this summary and its descriptions of certain embodiments of such formulations and compositions, and will be readily apparent to those skilled in the art having knowledge of the synthetic techniques described therewith. Such features, benefits and advantages will be apparent from the above as taken into conjunction with the accompanying examples, data, and all reasonable inferences to be drawn therefrom.

### Detailed Description of Certain Embodiments

The invention relates to a formulation for a cleaning composition (or simply a "cleaning composition") comprising at least one lactate compound, a glucose derivative, an alcohol, which in one exemplary embodiment is a diol and/or glycol, and a optional phospholipid complex. The cleaning composition of the invention can be used, for example, on personal care wet wipes such as baby wipes, facial wipes, moist toilet tissue, pet wipes, antibacterial wipes, hand-cleaning wipes and wipes for incontinence and feminine hygiene, as well as other types of cleaning wet wipes. As used herein, a "wipe" is a type of article suitable for cleansing or disinfecting or for applying a compound and/ or removing materials and compounds from skin and surfaces, such as those disclosed in US Patent Nos. 7,101,612; 6,814,974; and 6,444,214, each of which is hereby expressly incorporated by reference in its entirety for all purposes. In one particular embodiment of the disclosure, this term refers to an article for cleansing the body, including the removal of bodily waste.

Preferably, the optional phospholipid complex is an organic phospholipid. Non-limiting examples of organic phospholipids include alkyl PG-dimonium chloride phosphates with a fatty acid chain length between 8 and 20 carbons, such as, for example, cocamidopropyl PG-dimonium chloride phosphate. Other non-limiting examples of suitable alkyl PG-dimonium chloride phosphates include sodium coco PG-dimonium chloride phosphate, stearamidopropyl PG-dimonium chloride phosphate, myristalamidopropyl PG-dimonium chloride phosphate, sodium borageamidopropyl PG-dimonium chloride phosphate, linoleamidopropyl PG-dimonium chloride phosphate, linoleamidopropyl PG-dimonium chloride phosphate dimethicone, and the like.

Referring to the cocamidopropyl PG-dimonium chloride phosphate, specifically, the complex belongs to a family of products that are multifunctional, natural triglyceride phospholipids and are similar to phospholipids that occur naturally in the body. Cocamidopropyl PG-dimonium chloride phosphate is a coconut oil derived phospholipid composed predominantly of diester and triester phosphatides with multiple-chain groups. It displays a broad range of functional attributes including gentle cleansing and foaming properties, anti-irritation effects when combined with anionic or nonionic surfactants, unusually high substantivity, long-lasting skin conditioning, and broad spectrum antimicrobial activity. Cocamidopropyl PG-dimonium chloride phosphates generally have the following formula: 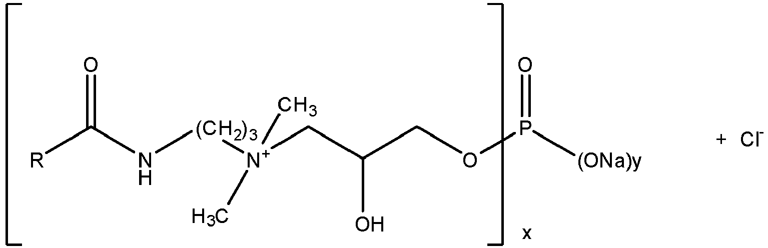 wherein the "R" group originates from coconut oil (i.e. C₈-C₂₀ or C₁₀-C₂₀ alkyl) and "x" + "y" equals the integer 3.

As referred to herein, "lactate" is an anion having the following structure: 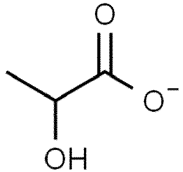 wherein the lactate anion forms a lactic acid salt when bonded to one of a number of various cations, including but not limited to zinc, calcium, potassium, and sodium, and combinations therefor, among others. Further, in one exemplary embodiment the lactic acid salt is zinc lactate which, while known as an antioxidant nutrient, in the present disclosure functions in an unexpected and synergistic manner with the other components of the preservative composition including a glucose derivative, a polyhydric alcohol, and an optional phospholipid complex, to provide the beneficial antimicrobial effects of the preservative formulation included with a cleaning composition on a wet wipe.

Another component of the preservative formulation is an alcohol, which can be one or more polyhydric alcohols that in one exemplary embodiment takes the form of a diol. In certain exemplary embodiments, one or more diols can be included in the composition, with the one or more diols present in an amount of between from about 0.1% to 10.0% by weight of the cleaning composition, or alternatively between about 0.3% to about 5.0% by weight of the cleaning composition. Further, each diol can be selected from diols having a primary chain length of C₂-C₂₀ in some exemplary embodiments, as well as diols having a primary chain length of C₃-C₁₂ in other exemplary embodiments, such as a 1,3-diol, such as 1,3-propanediol, or a 1,2-diol, such as 1,2-hexanediol, which has the following structure: 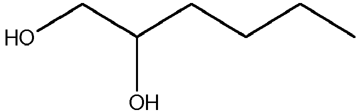 or, 1,5-pentanediol, which has the following structure:

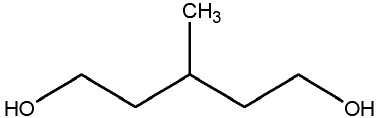

In addition to, or as a substitute for one or more of the polyhydric alcohols and/or diols, the composition can also include a glycol, alone or in combination with another diol. While a number of different glycols can be utilized, in one exemplary embodiment the glycol is propylene glycol and has the following general structure:

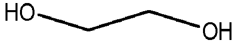

Other forms of a diol which can be useful as the polyhydric alcohol component in the preservative formulation incorporated within the cleaning composition described herein include molecules derived from glycerin or other triols. Glycerin and other triols can be reacted with other molecules to create a diol such examples such as glyceryl monoethers, such as ethylhexylglycerin [3-(2-ethylhexyloxy)propane-1,2-diol] and or glyceryl monoesters, such as glyceryl monolaurate, and glyceryl monocaprylate.

The preservative composition further includes a glucose derivative. In one exemplary embodiment, the glucose derivative is formed as a salt of gluconic acid, such as calcium gluconate and/or sodium gluconate which, while known as chelating agent, in the present disclosure functions in an unexpected and synergistic manner with the other components of the preservative composition to provide the antimicrobial function of the preservative composition.

It is to be understood that the cleaning composition including the preservative formulation described herein in one exemplary embodiment is an aqueous solution, with water forming the balance of the cleaning composition outside of the components of the preservative formulation. However, the cleaning composition can optionally include various other components or adjuncts. The lactic acid salt, the gluconic acid salt, the one or more diols and/or glycols, the optional alkyl PG-dimonium chloride phosphates and various other components are blended together to form a wet wipe cleaning composition, solution or emulsion that can be applied to a substrate to form a usable wet wipe that is sufficiently preserved for storage and consumer use.

The combination of the one or more lactic acid salts, the one or more gluconic acid salts, and one or more diols and/or glycols in combination with one another and optionally the phospholipid complex/organic phospholipid provides an synergistic and enhanced performing preservative formulation that provides antimicrobial properties that are unexpected in light of the known efficacy of each of the individual components for this purpose, which is highly dissimilar in purpose to the known functions for each component, i.e., zinc lactate as an antioxidant and sodium gluconate as a chelating agent.

In certain particular exemplary embodiments of the wet wipe cleaning composition including the preservative formulation of the present disclosure, the amount of lactic acid salt, e.g., the zinc lactate, in the cleaning composition applied to the wipe substrate ranges from about 0.01% to about 5.0% by weight of the cleaning composition, and from about 0.05% to about 2.0% by weight of the cleaning composition, and even more specifically from about 0.1% to about 1.0% by weight of the cleaning composition

In certain particular exemplary embodiments of the wet wipe cleaning composition including the preservative formulation of the present disclosure, the amount of gluconic acid salt, e.g., the sodium gluconate, in the cleaning composition applied to the wipe substrate ranges from about 0.01% to about 5.0% by weight of the cleaning composition, and more specifically from about 0.05% to about 2.0% by weight of the cleaning composition, and even more specifically from about 0.1% to about 1.0% by weight of the cleaning composition.

In another exemplary embodiment, the total amount of diol and/or glycol present in the preservative formulation within the wet wipe cleaning composition ranges from about 0.01% to about 10.0% by weight of the cleaning composition, and more specifically from about 0.3% to about 5.0% by weight of the cleaning composition, and even more specifically from about 0.5%. to about 3.0% by weight of the cleaning composition.

In still another exemplary embodiment, the amount of the optional organic phospholipid, e.g., the alkyl PG-dimonium chloride phosphate, in the wet wipe cleaning composition ranges from about 0.01% to about 2.0% by weight of the cleaning composition, and more specifically from about 0.1% to about 1.0% by weight of the cleaning composition.

As stated above, the cleaning composition may optionally include and/or be used in combination with one or more additional components or adjuncts. The adjuncts include, but are not limited to, water, surfactants, emollients, fragrances and/or perfumes, botanical extracts, oils and/or lotions, silicones, waxes, dyes and/or colorants, solubilizing materials, stabilizers, thickeners, defoamers, hydrotropes, chelating agents, buffers, builders, enzymes, solvents, bleaching agents, cloud point modifiers, preservatives and/or combinations of the same. Other anti-microbial components including pH adjusters can additionally be added to the preservative formulation and/or cleaning composition, including one or more organic salts, e.g., sodium citrate or sodium benzoate, one or more organic acids, e.g., citric acid, among others, and combinations thereof. Any suitable solvent can be utilized with the preservative formulation and cleaning composition including the preservative formulation of the invention, such as water as discussed above. In certain alternative, exemplary embodiments the solvent can be formed from water and/or C₂-C₂₀ diols, but preferably C₃-C₁₂ diols, or from C₂-C₂₀ but preferably C₃ - C₁₂ glycols/diols. Further, the diols and/or glycols utilized as the solvent or portions thereof can be the same as or different from those utilized in the preservative formulation, such that in certain embodiments the preservative formulation can function as the solvent.

In one exemplary embodiment of the present invention, the cleaning composition can be loaded onto an absorbent substrate. The absorbent substrate is preferably water-insoluble. By "water insoluble" it is meant that the substrate does not dissolve but may readily break apart upon immersion in water. This cleaning composition can be used on flushable wipes in which the nonwoven substrate readily breaks apart after flushing. The water insoluble substrate is the implement or vehicle for delivering the cleaning composition of the present invention to the skin to be cleansed and moisturized. As used herein, the terms "substrate" or "wipe" are intended to include any material on which a cleaning composition may be loaded. In functional applications, a substrate is used to clean an article or a surface, as by wiping. Substrates comprise woven or non-woven materials, typically made from a plurality of fibers. The substrate can be used by itself (typically by hand) or attached to a cleaning implement, such as a floor mop, handle, or a handheld cleaning tool, such as a toilet cleaning device. A wide variety of materials can be used as the substrate. Nonlimiting examples of suitable water insoluble substrates include nonwoven substrates, woven substrates, sponges, cloths, meshes, paper towels, napkins, cleaning pads, and the like.

Preferred embodiments employ nonwoven substrates since they are economical and readily available in a variety of materials. By nonwoven is meant that the layer is comprised of fibers which are not woven into a fabric but rather are formed into a membrane, sheet, substrate, mat, absorbent core or pad layer or combinations thereof. Nonwoven substrates may be comprised of a variety of materials both natural and synthetic. By "natural" it is meant that the materials are derived from plants, animals, insects or byproducts of plants, animals, and insects. By synthetic is meant that the materials are obtained primarily from various man-made materials or from natural materials which have been further altered. The conventional base starting material is usually a fibrous web comprising any of the common synthetic or natural textile-length fibers, or mixtures thereof. Non-limiting examples of natural materials useful in the present invention are silk fibers, keratin fibers and cellulosic fibers. Non-limiting examples of keratin fibers include those selected from the group consisting of wool fibers, camel hair fibers, and the like. Non-limiting examples of cellulosic fibers include those selected from the group consisting of wood pulp fibers, cotton fibers, hemp fibers, jute fibers, flax fibers, and mixtures thereof. Non-limiting examples of synthetic materials useful in the present invention include those selected from the group consisting of acetate fibers, acrylic fibers, cellulose ester fibers, modacrylic fibers, polyamide fibers, polyester fibers, polyolefin fibers, polyvinyl alcohol fibers, rayon fibers, polyurethane foam, and mixtures thereof. Examples of some of these synthetic materials include acrylics such as acrilan, creslan, and the acrylonitrile-based fiber, orlon; cellulose ester fibers such as cellulose acetate, arnel, and acele; polyamides such as nylons; polyesters such as fortrel, kodel, and the polyethylene terephthalate fiber, dacron; polyolefins such as polypropylene, polyethylene; polyvinyl acetate fibers; polyurethane foams and mixtures thereof. It is noted that the range of cleaning solution impregnated into the substrate includes from between 100% to 500% of the dry weight of the substrate.

Without being bound to any theory, the invention has shown that the lactic acid salt, the gluconic acid salt and the polyhydric alcohol alone provides a preservative booster to the preservative formulation, optionally boosted by the phospholipid complex (such as cocamidopropyl PG-dimonium chloride phosphate), depending on the level in the final preservative formulation.

### EXAMPLES

Table 1 shows multiple formulations for cleaning compositions to be loaded onto wet wipes, with Formulation A including components and amounts of the same that represent a specific working example of the inventive cleaning composition and preservative composition provided therein. The other Formulations B-D listed below are cleaning compositions including or replicating components of Formulation A, but one or more of the components of the preservative formulation which were developed and tested separately to illustrate the synergistic, unexpected and non-obvious antimicrobial properties of the preservative formulation and cleaning composition including the preservative formulation.

**TABLE 1. Wet Wipe Cleaning Composition Formulations**

| **Ingredient** | **% Use Level** | **% Level** | **Formulation A** | **Formulation B** | **Formulation C** | **Formulation D** | **Formulation E** |
|---|---|---|---|---|---|---|---|
| Water | 97 | 100 | 97.2675 | 99.428 | 99.2 | 98.3 | 99.6 |
| Cocamidopropyl PG Phosphate | 0.5 | 46.5 | 0.2325 | 0.372 | - | - | - |
| Zinc lactate | 0.6 | 100 | 0.6 | - | 0.6 | - | - |
| 1, 3-propanediol | 1.0 | 100 | 1.0 | - | - | 1.0 | - |
| Ethylhexylglycerin | 0.5 | 100 | 0.5 | - | - | - | - |
| Sodium gluconate | 0.2 | 100 | 0.2 | - | - | - | 0.2 |
| 1, 2-Hexanediol | 1.5 | 100 | - | | - | 1.5 | - |
| Sodium Citrate | 0.1 | 100 | 0.1 | 0.1 | 0.1 | 0.1 | 0.1 |
| Citric Acid | 0.1 | 100 | 0.1 | 0.1 | 0.1 | 0.1 | 0.1 |

To illustrate the efficacy of the preservative formulation and cleaning composition formed with the preservative composition of the present invention, a challenge test complying with USP/EP/BP methodologies was performed on the example Formulations A-E having the components listed in Table 1. More specifically, the testing performed on Formulations A-E of Table 1 to obtain the results shown in Table 2, was the PCPC M-5 test, Method for Preservation Test of Nonwovens Substrates Personal Care Products. In the test procedure, representative samples of each formulation were inoculated with separate mixed cultures of bacteria and fungi comprising of:
Inoculum A - Mixed Bacteria: *Pseud. aeruginosa* (ATCC 15442); *B. cepacia* (ATCC 25416); E*.coli* (ATCC 8739 or 11229); *S*. *aureus* (ATCC 6538).
Inoculum B - Mixed fungi: *A. brasiliensis* (ATCC 16404 - *niger)*; *C. albicans* (ATCC 10231); *Penicillium pinophilium* (ATCC 9644) or *Penicillium levitum* (ATCC 10464).

Table 2 shows the results from test and shows or indicates a log value of the number of viable organisms measured after the specified time interval from inoculation of the sample with the bacteria cultures. The term "TNTC" is an acronym for "Too Numerous To Count" and indicates the number of viable organisms has increased in comparison to the initial inoculum.

**TABLE 2. PCPC M-5 Test Results**

| | | **Initial (cfu/gram)** | **24hr (cfu/gram)** | **72hr (cfu/gram)** | **7 Day (cfu/gram)** | **14 day (cfu/gram)** | **Result Pass/Fail** |
|---|---|---|---|---|---|---|---|
| **Formulation A** | **Inoculum A** | 1,200,000 | <10 | <10 | <10 | <10 | **Pass** |
| | **Inoculum B** | 440,000 | <10 | <10 | <10 | <10 | |
| **Formulation B** | **Inoculum A** | 1,900,000 | TNTC | TNTC | TNTC | TNTC | **Fail** |
| | **Inoculum B** | 340,000 | 3,900 | 350 | <10 | 5,650 | |
| **Formulation C** | **Inoculum A** | 2,200,000 | 6,300 | 8,500 | TNTC | N/A | **Fail** |
| | **Inoculum B** | 430,000 | 29,000 | 42,000 | TNTC | N/A | |
| **Formulation D** | **Inoculum A** | 1,100,00 | 48,000 | 6,150 | <10 | 750 | **Fail** |
| | **Inoculum B** | 350,000 | 53,000 | 24,000 | 9,800 | 36,000 | |
| **Formulation E** | **Inoculum A** | 1,500,000 | TNTC | TNTC | N/A | N/A | **Fail** |
| | **Inoculum B** | 410,000 | TNTC | TNTC | N/A | N/A | |

The results from Table 2 show that the composition according to the invention example Formulation A demonstrates the only acceptable log reduction for a personal care/cleaning product across both sets of inoculum (A & B) compared to the other Formulations B-E that were tested each including certain components of the preservative composition used in the cleaning composition of the present invention, but not all of the components, which all failed under USP/EP/BP testing criteria, illustrating the synergistic effects of the combination of the components forming the preservative composition of the wet wipe cleaning composition.

The disclosures of all articles and references, including patents, are incorporated herein by reference. The invention and the manner and process of making and using it are now described in such full, clear, concise and exact terms as to enable any person skilled in the art to which it pertains, to make and use the same. All references cited in this specification are incorporated herein by reference. It is to be understood that the foregoing describes preferred embodiments of the present invention and that modifications may be made therein without departing from the spirit or scope of the present invention.

## Claims

1. A cleaning composition including a preservative formulation comprising a lactic acid salt, a gluconic acid salt and a polyhydric alcohol.

2. A cleaning composition according to claim 1, further comprising an organic phospholipid.

3. A cleaning composition according to claim 2, wherein the organic phospholipid is an alkyl PG-dimonium chloride phosphate having a fatty acid chain length between 10 and 20 carbons and/or wherein the organic phospholipid is present in the cleaning composition in an amount of from 0.1% to 2.0% by weight.

4. A cleaning composition according to any preceding claim, wherein the lactic acid salt is zinc lactate.

5. A cleaning composition according to any preceding claim, wherein the lactic acid salt is present in the cleaning composition in an amount of from about 0.01% to about 5.0% or from about 0.1% to about 1.0% by weight of the cleaning composition.

6. A cleaning composition according to any preceding claim, wherein the gluconic acid salt is sodium gluconate.

7. A cleaning composition according to any preceding claim, wherein the gluconic acid salt is present in the cleaning composition in an amount of from about 0.01% to about 5.0% by weight of the cleaning composition.

8. A cleaning composition according to claim 7, wherein the gluconic acid salt is present in the cleaning composition in an amount of from about 0.1% to about 1.0% by weight of the cleaning composition.

9. A cleaning composition according to any preceding claim, wherein the polyhydric alcohol is present in an amount of between from about 0.1% to 10.0% or between about 0.3% to about 5.0% by weight of the cleaning composition.

10. A cleaning composition according to any preceding claim, further comprising one or more adjuncts selected from a group consisting of water, anti-microbial components, surfactants, emollients, fragrances and/or perfumes, extracts, oils and/or lotions, silicones, waxes, dyes and/or colorants, solubilizing materials, stabilizers, thickeners, defoamers, hydrotropes, buffers, builders, enzymes, bleaching agents, cloud point modifiers, and preservatives.

11. A cleaning wipe comprising:
a. a substrate; and
b. a cleaning composition loaded onto the substrate, the cleaning composition comprising a preservative formulation comprising a lactic acid salt, a gluconic acid salt and a polyhydric alcohol.

12. A cleaning wipe according to claim 11, wherein:
the lactic acid salt is zinc lactate;
the gluconic acid salt is sodium gluconate; and/or
the polyhydric alcohol is a diol.

13. A cleaning wipe according to claim 11, wherein the preservative formulation further comprises an organic phospholipid.

14. A method of cleaning a surface, the method comprising the steps of:
a) providing a cleaning composition comprising a preservative formulation including a lactic acid salt, a gluconic acid salt and a polyhydric alcohol; and
b) applying the cleaning composition to surface.

15. A method according to claim 14, wherein the step of providing the cleaning composition comprises the step of applying the cleaning composition to a substrate to form a cleaning wipe.
